(19) Europäisches Patentamt
European Patent Office
Office européen des brevets

(11) Numéro de publication: 0 169 755
B1

(12) # FASCICULE DE BREVET EUROPEEN

(45) Date de publication du fascicule du brevet: 09.05.90

(21) Numéro de dépôt: 85401233.3

(22) Date de dépôt: 20.06.85

(51) Int. Cl.[5]: C 07 D 417/12, C 07 D 417/14, C 07 D 401/12, C 07 D 401/14, C 07 D 413/12, C 07 D 413/14, A 61 K 31/47, A 61 K 31/505, C 07 D 265/22, C 07 D 277/46 // C07D413/06

(54) **Nouveaux dérivés de l'acide 4-hydroxy 3-quinoléine carboxylique subsitités en 2 par une fonction aminée, leur préparation, leur application comme médicaments, les compositions les renfermant et les intermédiaires nouveaux obtenus.**

(30) Priorité: 25.06.84 FR 8409960

(43) Date de publication de la demande: 29.01.86 Bulletin 86/05

(45) Mention de la délivrance du brevet: 09.05.90 Bulletin 90/19

(84) Etats contractants désignés: AT BE CH DE FR GB IT LI LU NL SE

(56) Documents cités:
DE-A-3 320 102
FR-A-2 532 939

Le dossier contient des informations techniques présentées postérieurement au dépôt de la demande et ne figurant pas dans le présent fascicule.

(73) Titulaire: ROUSSEL-UCLAF
35, boulevard des Invalides
F-75007 Paris (FR)

(72) Inventeur: Clemence, François
2, rue Turgot
F-75009 Paris (FR)
Inventeur: Le Martret, Odile
42, avenue de Versailles
F-75016 Paris (FR)
Inventeur: Delevallee, Françoise
48-50, avenue de la Dame Blanche
F-94120-Fontenay Sous Bois (FR)

(74) Mandataire: Fritel, Hubert et al
Département des Brevets ROUSSEL UCLAF B.P. no 9
F-93230 Romainville (FR)

EP 0 169 755 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne de nouveaux dérivés de l'acide 4-hydroxy-3-quinoléine carboxylique substitués en 2 par une fonction aminée, leur préparation, leur application comme médicaments, les compositions les renfermant et les intermédiaires nouveaux obtenus.

Des dérivés similaires à ceux de la présente invention mais portant en 2 soit une fonction soufrée soit une fonction hydroxylée sont décrits dans les brevets français 2532939 et allemand 3320102.

Cependant, ces produits présentent soit une moins bonne activité anti-inflammatoire soit une moins bonne tolérance gastrique que les produits de la demande.

L'invention a pour objet les composés de formule (I):

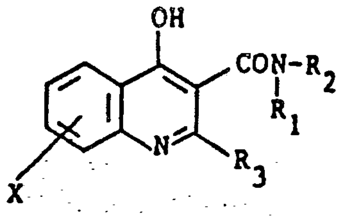

(I)

dans laquelle X en position 5, 6, 7 ou 8 représente un atome d'hydrogène, un atome d'halogène, un radical alcoyle liniéaire ou ramifié renfermant de 1 à 5 atomes de carbone, un radical alcoxy linéaire ou ramifié renfermant de 1 à 4 atomes de carbone, un radical trifluorométhyle, un radical trifluorméthylthio ou un radical trifluorométhoxy, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle et tétrazolyle, éventuellement substitués par un radical alcoyle renfermant de 1 à 4 atomes de carbone et phényle, éventuellement substitué par un ou plusieurs radicaux choisis dans le groupe formé par le radical hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène, $R_3$ représente un radical 2-pyrrolidinyle de formule

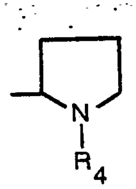

ou un radical —

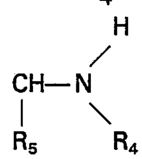

dans lesquels $R_4$ représente soit un atome d'hydrogène, soit un groupe

$$-\underset{\underset{O}{\|}}{C}-R'_4,$$

$R'_4$ étant un radical alcoyle renfermant de 1 à 5 atomes de carbone éventuellement substitué par un radical amino libre ou protégé, un radical phényle ou benzyle, soit un radical protecteur de la fonction amino, $R_5$ représente un atome d'hydrogène, un radical alcoyle renfermant de 1 à 5 atomes de carbone, un radical phényle ou benzyle, ou un radical 4—OH benzyle, un radical 1H-indol 3-yl méthyl, de formule:

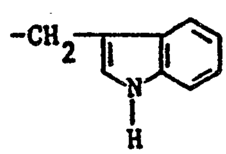

un radical

$$-CH_2OH \text{ ou } -\underset{\underset{OH}{|}}{CH}-CH_3 \text{ ou } -CH_2SH,$$

les fonctions alcool et thiol de ces trois derniers radicaux étant éventuellement bloquées par des groupes protecteurs, sous leurs formes racémiques ou optiquement actives, ainsi que leurs sels d'addition avec les acides.

Il va de soi que les terms "racémiques ou optiquement actifs" ne concernent que les composés de formule (I) qui comportent au moins un carbone asymétrique.

Lorsque X représente un atome d'halogène, il s'agit de préférence d'un atome de chlore.

Lorsque X représente un radical alcoyle, il s'agit de préférence du radical méthyle, éthyle, n-propyle, n-butyle, n-pentyle, isopropyle ou isobutyle.

Lorsque X représente un radical alcoxy, il s'agit de préférence du radical méthoxy, éthoxy ou n-propoxy.

Lorsque $R_1$ représente un radical alcoyle, il s'agit de préférence du radical méthyle ou éthyle.

Lorsque $R_2$ représente un radical hétérocyclique substitué par un radical alcoyle, il s'agit de préférence d'un radical hétérocyclique substitué par un radical méthyle ou éthyle.

Lorsque $R_2$ représente un radical phényle substitué, il s'agit de préférence d'un radical phényle substitué par un ou plusieurs radicaux choisis dans le groupe formé par le radical hydroxy, les radicaux méthyle et éthyle, les radicaux méthoxy et éthoxy, le radical trifluorométhyle, le radical nitro et l'atome de chlore.

Lorsque $R_4$ représente

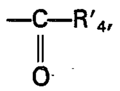

$R'_4$ est de préférence un radical alcoyle, par exemple le radical méthyle, éthyle, n-propyle, isopropyle, n-butyle, isobutyle ou n-pentyle.

Par groupement protecteur de la fonction amino, on entend groupement que l'on peut éliminer facilement, par exemple par hydrolyse ou réduction.

Des radicaux qui sont capables de protéger de façon efficace un radical amino sont généralement des radicaux acides par exemple un radical acide dérivant d'un acide carboxylique aliphatique, aromatique, araliphatique ou hétérocyclique tel que l'acide acétique, l'acide benzoïque ou l'acide pyridine-carboxylique ou un radical acide dérivant d'un acide carbonique tel que le radical éthoxy-carbonyle, benzyloxycarbonyle (=Z), tert-butyloxy-carbonyle (=Boc) ou p-méthyloxy-benzyloxy-carbonyle, ou un radical acyle dérivant d'un acide sulfonique tel que le radical benzènesulfonyle ou p-toluènesulfonyle, mais on peut également utiliser d'autres radicaux tels que des radicaux aryle ou aralkyle substitués ou non, par exemple les radicaux benzyle et triphénylméthyle ou des radicaux tels que les radicaux o-nitrophényl sulfényle et 2-benzoyl-1-méthylvinyle.

Lorsque $R_5$ représente un radical alcoyle il s'agit de préférence d'un des radicaux mentionnés précédemment pour $R'_4$.

Les groupements protecteurs de la fonction thiol sont par exemple les groupements benzyle, benzyloxycarbonyle, tert-butyloxycarbonyle.

Les groupements protecteurs de la fonction alcool sont par exemple les groupements benzyle, tert-butyle, benzyloxycarbonyle.

Parmi les sels d'additon avec les acides, on peut citer ceux formés avec les acides minéraux tels que les acides chlorhydrique, bromohydrique, sulfurique et phosphorique ainsi que ceux formés avec les acides sulfoniques tels que les acides alcoyl ou arylsulfoniques, par exemple l'acide méthanesulfonique ou paratoluènesulfonique.

L'invention a notamment pour objet les composés de formule (I) pour lesquels X est en position 8 et ceux pour lesquels X représente un radical trifluorométhyle, sous leurs formes racémiques ou optiquement actives, ainsi que leurs sels d'addition avec les acides et ceux pour lesquels $R_1$ représente un atome d'hydrogène et pour lesquels $R_2$ représente le radical thiazolyle, sous leurs formes racémiques ou optiquement actives, ainsi que leurs sels d'addition avec les acides.

L'invention a plus particulièrement pour objet les composés de formule (I) pour lesquels les radicaux protecteurs de la fonction amino sont des groupements de formule $—CO_2A$, A étant un radical alcoyle renfermant de 1 à 5 atomes de carbone, un radical aryle ou arylalcoyle, sous leurs formes racémiques ou optiquement actives, ainsi que leurs sels d'addition avec les acides et notamment ceux pour lesquels A est un radical terbutyle ou benzyle, sous leurs formes racémiques ou optiquement actives, ainsi que leurs sels d'addition avec les acides.

L'invention concerne tout particulièrement les composés de formule (I) pour lesquels $R_4$ représente un atome d'hydrogène, sous leurs formes racémiques ou optiquement actives, ainsi que leurs sels d'addition avec les acides et ceux se présentant sous leur forme optiquement active (S), ainsi que leurs sels d'addition avec les acides.

L'invention a aussi pour objet un procédé de préparation des produits de formule (I), dans laquelle X, $R_1$, $R_2$ et $R_3$ ont les significations données précédemment, caractérisé en ce que l'on soumet un composé de formule (II):

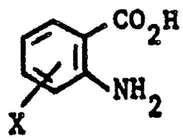

(II)

dans laquelle X conserve la signification donnée ci-dessus, à l'action d'un acide aminé de formule (III):

$R_3$—COOH (III)

sous forme activée et sous forme racémique ou optiquement active, dans lequel $R_3$ est un groupe:

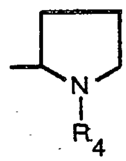

ou un groupe —

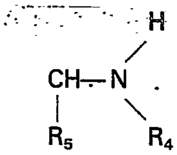

$R_4$ étant soit un groupe

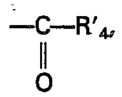

$R'_4$ ayant la signification donnée précédemment, soit un radical protecteur de la fonction amino, $R_5$ ayant la signification donnée précédemment, étant entendu que lorsque $R_5$ représente un groupe

$-CH_2OH$ ou 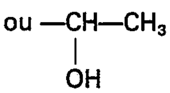 ou $-CH_2SH$

et lorsque $R'_4$ représente un radical alcoyle substitué par un radical amino, les fonctions alcool, thiol et amine, sont éventuellement bloquées, pour obtenir un composé de formule (IV), sous forme racémique ou optiquement active:

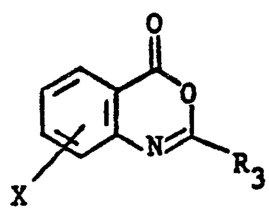

(IV)

dans laquelle X et $R_3$ conservent leurs significations précédentes, que l'on soumet, en présence d'une base, à l'action d'un composé de formule (V):

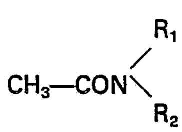

(V)

dans laquelle $R_1$ et $R_2$ conservent leurs significations précédentes, puor obtenir un composé de formule (VI) sous forme racémique ou optiquement active:

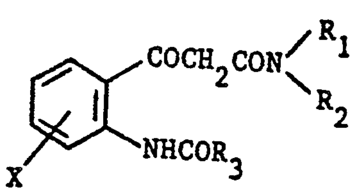

(VI)

dans laquelle X, $R_1$, $R_2$ et $R_3$ conservent les significations précédentes, que l'on cyclise en présence d'un agent alcalin, pour obtenir un composé de formule (I) dans laquelle X, $R_1$, $R_2$ et $R_3$ ont les significations qui viennent d'être données, sous forme racémique ou optiquement active, que l'on soumet, le cas échéant, à l'action d'un ou plusieurs agents de déblocage des fonctions amino, alcool et thiol, pour obtenir un produit de formule (I) dans laquelle X, $R_1$, et $R_2$ ont les significations précédentes et dans laquelle $R_3$ représente soit un groupe:

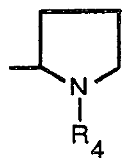

soit un groupe

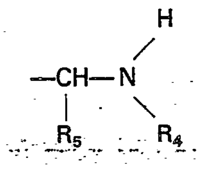

dans lesquels $R_4$ est un groupe

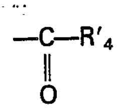

ou un atome d'hydrogène et $R_5$ a la signification précédente, étant entendu que lorsque $R_5$ représente un groupement

$CH_2OH$, 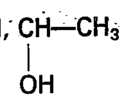 ou $CH_2SH$

et lorsque $R'_4$ représente un radical alcoyle substitué par un radical amino, les fonctions alcool, thiol et amine sont libres, produits de formule (I), sous forme racémique ou optiquement active, que l'on soumet, si désiré, à l'action d'un acide pour en former le sel.

Dans un mode de réalisation préferée du procédé de l'invention:

— Le procédé d'activation de l'acide aminé de formule (III) est un procédé non racémisant. On utilise avantageusement l'acide aminé de formule (III) activé sous forme d'anhydride mixte à l'aide du chloroformiate d'isobutyle mais d'autres chloformiates d'alcoyle peuvent être utilisés.

L'activation de l'acide aminé peut se faire par d'autres méthodes d'amidification connues en synthèse peptidique, comme par exemple la methode utilisant le dicyclohexylcarbodiimide activé par l'hydroxybenzotriazole.

— La réaction entre le produit de formule (II) et le produit de formule (III) s'effectue en présence d'une base comme par exemple la N-méthyl morpholine, la N-éthyl morpholine, la triéthylamine, à une température se situant de préférence entre −20°C et 0°C.

— La réaction entre le composé de formule (IV) et le composé de formule (V) a lieu en présence d'une base qui est notamment un organolithien comme le n-butyllithium ou un amidure de lithium tel que le diisopropylamidure de lithium. Cette réaction s'effectue de préférence à une basse température de l'ordre de −70°C.

— La cyclisation du composé de formule (VI) s'effectue en présence d'un agent alcalin, tel qu'un hydrure ou un carbonate alcalin, ou d'une amine, par exemple d'hydrure de sodium, de carbonate de sodium ou de potassium, de pipéridine, de 4-aminopyridine, de diméthyl aminopyridine, de triéthylamine, de 1,5-diazabicyclo /4,3,0/ non-5-ène, de 1,4-diazabicyclo /2,2,2/ octane ou de 1,5-diazabicyclo /5,4,0/ undec-5-ène.

— Les agents de déblocage des fonctions amino, alcool et thiol sont variables suivant les groupes protecteurs utilisés.

Lorsque le groupe protecteur est le groupe Boc, le déblocage s'effectue par hydrolyse acide, notamment en présence d'acide trifluoroacétique ou d'acide chlorhydrique ou encore par action du tribromure de bore.

Si le groupe protecteur utilisé est le groupe Z ou benzyle, le déblocage s'effectue par hydrogénation catalytique.

L'invention a également pour objet une variante du procédé tel que décrit précédemment, pour la

préparation des produits de formule (I) dans laquelle X, $R_1$ et $R_2$ ont les significations données précédemment et $R_3$ représente un radical

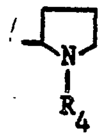 ou 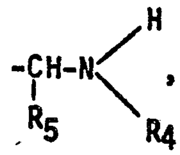 ,

dans lequel $R_4$ représente un groupe

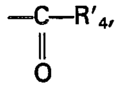

$R'_4$ étant un radical alcoyle renfermant de 1 à 5 atomes de carbone, éventuellement substitué par un radical amino libre ou protégé, un radical aryle ou aralcoyle, caractérisé en ce que l'on fait réagir un produit de formule (I), dans laquelle $R_3$ représente un radical 2-pyrrolidinyle ou un radical

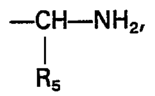

sous forme racémique ou optiquement active, avec un dérivé réactif du groupement

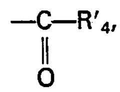

pour obtenir un produit de formule (I) correspondant, sous forme racémique ou optiquement active, que l'on soumet, le cas échéant, à l'action d'un agent de déblocage de la fonction amino, produit de formule (I), que l'on soumet, si désiré, à l'action d'un acide pour en former le sel.

Dans un mode de réalisation préférée du procédé de l'invention:

— Le dérivé réactif du groupement

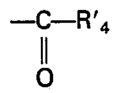

est un anhydride ou un halogénure et l'on opère en présence d'une base telle que la pyridine ou la N-méthyl morpholine.

— L'agent de déblocage de la fonction amino est l'un de ceux mentionnés plus haut.

Les composés de formule (I) tels que définis ci-dessus ainsi que leurs sels d'addition avec les acides présentent d'intéressantes propriétés pharmacologiques. Ils présentent en particulier, une très bonne activité analgésique ainsi qu'une activité anti-inflammatoire non négligeable.

Ces propriétés justifient leur application en thérapeutique et l'invention a également pour objet à titre de mèdicaments, les produits tels que définis par la formule (I) ci-dessus, ainsi que les sels d'addition avec les acides pharmaceutiquement acceptables desdits produits de formule (I).

L'invention a plus particulièrement pour objet, à titre de médicaments les composés de formule (I) pour lesquels $R_4$ représente un atome d'hydrogène, sous leurs formes racémiques ou optiquement actives, ainsi que leurs sels d'addition avec les acides pharmaceutiquement acceptables, ainsi que les composés de formule (I) sous leur forme optiquement active (S), ainsi que leurs sels d'addition avec les acides pharmaceutiquement acceptables.

Les médicaments, objets de l'invention, peuvent être préconisés dans le traitement des maladies inflammatoires dégénératives telles que l'ostéoarthrose, les collagénoses diverses (tendinites), des maladies rhumatismales (la polyarthrite rhumatoïde, spondylarthrite enkylosante), ainsi que dans le traitement d'autres maladies de nature auto-immune telles que le lupus érythémateux disséminé, les glomérulonéphrites et la sclérose en plaques.

Les médicaments, objets de l'invention, peuvent également être utilisés dans le traitement des algies musculaires, articulaires ou nerveuses, les douleurs dentaires, les migraines, le zona et également, à titre de traitement complémentaire dans les états infectieux et fébriles.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples our dragéifiés, les gélules, les granulés, les suppositoires, les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, la lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif par jour, par voie orale.

Les composés de formule (II), utilisés comme produits de départ d'un des procédés de l'invention sont des produits connus d'une façon générale, qui peuvent être préparés selon les procédés indiqués dans la demande de brevet français n° 2 157 874.

Les composés de formule (VI) sont des produits chimiques nouveaux; l'invention a donc pour objet ces produits, à titre de produits industriels nouveaux.

## Exemple 1

N-//4-hydroxy-8-(trifluorométhyl)-3-/(2-thiazolylamino) carbonyl/ 2-quinoléinyl/ méthyl/ carbamate de 1,1-diméthyl éthyle

*Stade A:* /(4-oxo 8-(trifluorométhyl) 4H-3,1-benzoxazin-2-yl) mèthyl/ carbamate de 1,1-diméthyl éthyle.

On mélange 17,5 g de N—BOC glycine, 100 cm3 de chlorure de méthylène et 13,7 cm3 de N-méthyl morpholine, refroidit à −20°C et ajoute 13 cm3 de chloroformiate d'isobutyle en solution dans 50 cm3 de chlorure de méthylène. On agite 30 minutes à −20°C et ajoute une solution renfermant 10,25 g d'acide 2-amino 3-trifluorométhyl benzoïque préparé selon le procédé indiqué dans J. Med. Chem. 16 (2) 101,6 (1973) dans 5,49 cm3 de N-méthyl morpholine et 120 cm3 de chlorure de méthylène. On agite 20 heures à température ambiante, verse sur 500 cm3 d'eau et 150 cm3 d'acide chlorhydrique 2N, extrait au chlorure de méthylène, lave à l'eau, séche, concentre sous pression réduite. Le résidu est empâté dans 50 cm3 de méthanol, glacé, essoré, lavé au méthanol; séché sous pression réduite à 50°C. On obtient 11 g de produit attendu fondant à 176°C.

*Analyse*

| | | | | |
|---|---|---|---|---|
| Calculé: | C% 52,33 | H% 4,39 | N% 8,14 | F% 16,55 |
| Trouvé: | 52,5 | 4,4 | 7,9 | 16,7 |

*Stade B:* N-/2-//2-/1,3-dioxo-3-(2-thiazolylamino) propyl/ 6-trifluorométhylphényl/ amino/ 2-oxo-éthyl/ carbamate de 1,1-diméthyle éthyle.

On introduit à 0°C, 85,6 cm3 de n-butyllithium en solution dans l'hexane, dans 8,5 g de N-2-thiazolyl acétamide en suspension dans 260 cm3 de tétrahydrofuranne. On refroidit à −75°C et ajoute à cette température, la solution de 10,33 g de produit obtenu au stade A précédent dans 75 cm3 de tétrahydrofuranne. On verse le mélange réactionnel sur 400 cm3 d'eau et 100 cm3 d'acide chlorhydrique 2N, extrait à l'éther, lave la phase organique à l'acide chlorhydrique N, puis à l'eau, sèche, concentre sous pression réduite. On obtient une huile orange qui cristallise. Ono empâte le résidu dans l'éther, essore, lave à l'éther, sèche sous pression réduite et obtient 7,7 g de produit attendu fondant à 166°C.

*Analyse*

| | | | | | |
|---|---|---|---|---|---|
| Calculé: | C% 49,38 | H% 4,36 | N% 11,52 | F% 11,72 | S% 6,59 |
| Trouvé: | 49,2 | 4,3 | 11,3 | 11,7 | 6,5 |

*Stade C:* N-//4-hydroxy 8-(trifluorométhyl) 3-/(2-thiazolylamino) carbonyl/ 2-quinoléinyl/ méthyl/ carbamate de 1,1-diméthyl éthyle.

On agite 1 heure à température ambiante 7,3 g de produit obtenu au stade B précédent, 140 cm3 de tétrahydrofuranne et 2,4 g de 4-diméthylamino-pyridine. On élimine le solvant sous pression réduite, reprend le résidu par 100 cm3 d'eau et 20 cm3 d'acide chlorhydrique N, extrait avec un mélange d'acétate d'éthyle et de tétrahydrofuranne. On lave à l'eau, sèche, concentre le filtrat sous pression réduite. On obtient 7 g de produit brut que l'on empâte dans l'éther. On essore, lave à l'éther, sèche sous pression réduite à 60°C et obtient 6,6 g de produit attendu fondant à 238°C.

*Analyse*

| | | | | | |
|---|---|---|---|---|---|
| Calculé: | C% 51,28 | H% 4,09 | N% 11,96 | F% 12,17 | S% 6,84 |
| Trouvé: | 51,1 | 4,0 | 11,8 | 11,9 | 6,9 |

Exemple 2

2-(aminométhyl) 4-hydroxy N-(2-thiazolyl) 8-trifluorométhyl 3-quinoléine carboxamide.

On agite 1 heure à température ambiantes 6,3 g de produit obtenu à l'exemple 1, 125 cm3 de chlorure de méthylène et 63 cm3 d'acide trifluoroacétique. On élimine les solvants sous pression réduite. On reprend le résidu par 100 cm3 d'eau, amène le pH à 7 avec une solution aqueuse saturée de carbonate acide de sodium. On essore, lave à l'eau, sèche sous pression réduite à 100°C et obtient 4,8 g de produit attendu fondant vers 280°C.

*Analyse*

| | | | | | |
|---|---|---|---|---|---|
| Calculé: | C% 48,91 | H% 3,01 | N% 15,21 | F% 15,47 | S% 8,70 |
| Trouvé: | 49,0 | 3,2 | 15,3 | 15,1 | 8,6 |

Exemple 3

N-/(R) 1-/4-hydroxy 3-/(2-thiazolylamino) carbonyl/ 8-trifluorométhyl 2-quinoléinyl/ éthyl/ carbamate de 1,1-diméthyléthyle.

*Stade A:* N-/(R) 1-/4-oxo 8-(trifluorométhyl) 4H,3,1-benzoxazin-2-yl/ éthyl/ carbamate de 1,1-diméthyl éthyle.

On mélange 18,9 g de BOC—D-alanine, 25 cm3 de N-méthyl morpholine et 200 cm3 de chlorure de méthylène, refroidit à −20°C et ajoute 26 cm3 de chloroformiate d'isobutyle en solution dans 100 cm3 de chlorure de méthylène. On maintient à −20°C la suspension ainsi obtenue et ajoute 20,5 g d'acide 2-amino 3-trifluorométhyl benzoïque dans 10 cm3 de morpholine et 200 cm3 de chlorure de méthylène. On laisse revenir à température ambiante et agite 2 heures, verse sur 400 cm3 d'acide chlorhydrique, lave la phase chlorméthylénique dans 250 cm3 d'une solution aqueuse de carbonate de sodium à 10%, puis à l'eau, sèche et concentre sous pression réduite. On obtient 17,6 g de produit attendu fondant à 175°C.

$/\alpha/_D = +68° \pm 2°$ (c=0.8% $CH_3COOH$)

*Stade B:* N-/(R) 2-//2/1,3-dioxo 3-(2-thiazolylamino) propyl/ 6-(trifluorométhyl) phényl/ amino/ 1-méthyl 2-oxo éthyl/ carbamate de 1,1-diméthyl éthyle.

On ajoute entre −3° et −1°C, sous agitation et sous azote, 128,6 cm3 d'une solution de n-butyllithium dans une solution renfermant 12,78 g de N-(2-thiazolyl) acétamide dans 370 cm3 de tétrahydrofuranne. Après 15 minutes, cette solution est refroide à −70°C et on ajoute à cette température 10,75 g de produit obtenu au stade précédent dans 75 cm3 de tétrahydrofuranne. Après 30 minutes à −70°C, on verse sur 140 cm3 d'acide chlorhydrique N, extrait à l'acétate d'éthyle, sèche, concentre sous pression réduite. On reprend le résidu par 350 cm3 d'éther et 250 cm3 d'acide chlorhydrique N, concentre sous pression réduite. On obtient 14,1 g de produit attendu.

*Stade C:* N-/(R)1-/4-hydroxy 3-/(2-thiazolylamino) carbonyl/ 8-trifluorométhyl 2-quinoléinyl/ éthyl/ carbamate de 1,1-diméthyl éthyle.

On laisse en contact 2 jours à température ambiante 13,8 g de produit obtenu au stade B précédent, 3,8 g de diméthylaminopyridine, 120 cm3 de tétrahydrofuranne, chasse le tétrahydrofuranne, reprend le résidu par 40 cm3 d'acide chlorhydrique N, agite 10 minutes, essore et lave à l'eau. Le produit brut est purifié par traitement au reflux, sans dissolution totale, dans 500 cm3 de méthanol. On obtient après refroidissement 9,4 g de produit attendu fondant à 265°C.

$/\alpha/_D = -6,5° \pm 1,5°$ (c=1% dans l'acide acétique).

*Analyse*

| | | | | | |
|---|---|---|---|---|---|
| Calculé: | C% 52,27 | H% 4,39 | N% 11,61 | F% 11,81 | S% 6,64 |
| Trouvé: | 52,4 | 4,4 | 11,5 | 11,7 | 6,8 |

Exemple 4

2-/(R) 1-aminoéthyl/ 4-hydroxy N-(2-thiazolyl) 8-trifluorométhyl 3-quinoléine carboxamide.

On met en suspension dans 140 cm3 de chlorure de méthylène, 7 g de produit obtenu à l'exemple 3. On ajoute peu à peu 70 cm3 d'acide trifluoroacétique, la température s'abaissant à 15°C. On maintient le contact pendant 4 heures, concentre sous pression réduite, reprend le résidu par 100 cm3 d'éther et essore les cristaux. Le produit obtenu est ajouté peu à peu à 50 cm3 d'une solution aqueuse de carbonate acide de sodium à 10%. On agite, essore, lave à l'eau. Le produit brut ainsi obtenu est traité au reflux par 300 cm3 de méthanol, sans dissolution complète. On refroidit, concentre jusqu'à 50 cm3 et obtient ainsi 2,3 g de produit attendu fondant à 265°C.

$/\alpha/_D = -72° \pm 2,5°$ (c=0,5% $CH_3COOH$).

*Analyse*

| | | | | | |
|---|---|---|---|---|---|
| Calculé: | C% 50,26 | H% 3,43 | F% 14,90 | S% 8,39 | N% 14,73 |
| Trouvé: | 50,3 | 3,6 | 15,0 | 8,4 | 14,5 |

Exemple 5

N-/(S) 1-/4-hydroxy 3-/(2-thiazolylamino) carbonyl/ 8-trifluorométhyl 2-quinoléinyl/ éthyl/ carbamate de 1,1-diméthyl éthyle.

*Stade A:* N-:(S) 1-/4-oxo 8-(trifluorométhyl) 4H-3,1-benzoxazin-2-yl/ éthyl/ carbamate de 1,1-diméthyl éthyle.

On opère dans des conditions identiques et sur la même unité opératoire que pour l'antipode (R) prèparé au stade A de l'exemple 3, à partir de Boc-L-alanine, on obtient 21,4 g de produit attendu fondant à 174°C.

$/\alpha/_D=-66,5°\pm1,5°$ (c=1% $CH_3COOH$).

*Analyse*

| | | | | |
|---|---|---|---|---|
| Calculé: | C% 53,63 | H% 4,78 | N% 7,82 | F% 15,90 |
| Trouvé: | 54,1 | 5 | 7,6 | 15,6 |

*Stade B:* N-/(S) 2-//2-/1,3-dioxo 3-(2-thiazolylamino) propyl/ 6-(trifluorométhyl) phényl/ amino/ 1-méthyl 2-oxo éthyl/ carbamate de 1,1-diméthyl éthyle.

On opère dans des conditions identiques et sur la même unité opératoire que pour l'antipode (R) préparé au stade B de l'exemple 3. On obtient 14,1 g de produit attendu.

*Stade C:* N-/(S) 1-/4-hydroxy 3-/2-thiazolylamino) carbonyl/ 8-trifluoromèthyl 2-quinoléinyl/ éthyl/ carbamate de 1,1-diméthyl éthyle.

On opère dans les mêmes conditions que pour l'antipode (R) préparé au stade C de l'exemple 3 à partir de 13,4 g de produit obtenu au stade B précédent, de 3,7 g de 4-diméthylaminopyridine et de 120 cm3 de tétrahydrofuranne. Le produit est purifié par traitement au reflux dans 700 cm3 de méthanol. On obtient 6,6 g de produit attendu fondant à 265°C.

$/\alpha/_D=+61°\pm2,5°$ (c=0,5% dans l'acide acétique).

*Analyse*

| | | | | | |
|---|---|---|---|---|---|
| Calculé: | C% 52,27 | H% 4,39 | N% 11,61 | F% 11,81 | S% 6,64 |
| Trouvé: | 52,4 | 4,4 | 11,4 | 11,7 | 6,9 |

Exemple 6

2-/(S) 1-aminoéthy/ 4-hydroxy N-(2-thiazolyl) 8-trifluorométhyl 3-quinoléine carboxamide.

On utilise le même procédé que celui utilisé pour préparer le produit de l'exemple 4, à partir de 4,95 g de produit de l'exemple 5 et obtient 4,1 g de produit brut. Le produit brut est purifié par traitement au reflux dans 360 cm3 de méthanol. On essore à chaud les cristaux obtenus et obtient 1,5 g de produit attendu fondant à 270°C.

$/\alpha/_D=+62°\pm3°$ (c=0,3% dans l'acide acétique).

*Analyse*

| | | | | | |
|---|---|---|---|---|---|
| Calculé: | C% 50,26 | H% 3,63 | F% 14,90 | S% 8,39 | N% 14,73 |
| Trouvé: | 50,1 | 3,4 | 15 | 8,5 | 14,4 |

Exemple 7

N-/(S)1-/4-hydroxy 3-/(2-thiazolylamino) carbonyl/ 8-trifluorométhyl 2-quinoléinyl/ propyl/ carbamate de 1,1-diméthyl éthyle.

*Stade A:* N-/(S) 1-/4-oxo 8-(trifluorométhyl)4H-3,1-benzoxazin-2-yl/ propyl/ carbamate de 1,1-diméthyle éthyle.

On refroidit à −20°C, une solution contenant 10,16 g d'acide BOC—L-amino butyrique dans 100cm3 de chlorure de méthylène et 13,7 cm3 de N-méthyl morpholine. On introduit à cette température 13 cm3 de chloroformiate d'isobutyle et 50 cm3 de chlorure de méthylène, agite 30 minutes entre −20°C et −15°C, ajoute à −20°C 10,25 g d'acide 2-amino 3-trifluorométhyl benzoïque en solution dans 100 cm3 de chlorure de méthylène et 5,49 cm3 de N-méthyl morpholine. On agite 20 heures à température ambiante, verse le mélange sur 200 cm3 d'acide chlorhydrique N, extrait au chlorure de méthylène, lave à l'eau puis au carbonate acide de sodium, à l'eau de nouveau, sèche et concentre le filtrat sous pression réduite. On empâte le résidu obtenu dans l'isopropanol, essore, lave à l'isopropanol, sèche sous pression réduite à 70°C et obtient 9,9 g de produit attendu fondant à 136—138°C.

$/\alpha/_D=-68°\pm1,5°$ (c=1% $CH_3COOH$).

*Analyse*

| | | | | |
|---|---|---|---|---|
| Calculé: | C% 54,84 | H% 5,14 | N% 7,52 | F% 15,31 |
| Trouvé: | 54,9 | 5,2 | 7,2 | 15,4 |

*Stade B:* N-/(S) 2-//2-/1,3-dioxo 3-(2-thiazolylamino) propyl/ 6-(trifluorométhyl) phényl/ amino/ 1-éthyl 2-oxo éthyl/ carbamate de 1,1-diméthyl éthle.

On opère comme indiqué au stade B de l'exemple 3 à partir de 7,78 g de N-(2-thiazolyl) acétamide, de 240 cm3 de tétrahydrofuranne, de 78,3 cm3 d'une solution de n-butyllithium dans l'hexane et de 10,2 g de produit obtenu au stade A précédent dans 75 cm3 de tétrahydrofuranne. On obtient 8,3 g de produit attendu fondant à 190°C.

$/\alpha/_D = -36{,}5 \pm 2°$ (c=0,5% $CH_3COOH$).

*Stade C:* N-/(S) 1-/4-hydroxy 3-/(2-thiazolylamino) carbonyl/ 8-trifluorométhyl 2-quinoléinyl/ propyl/ carbamate de 1,1-diméthyl éthyle.

On opère comme indiqué au stade C de l'exemple 3 à partir de 8 g de produit obtenu au stade B précédent, de 80 cm3 de tétrahydrofuranne et de 1,9 g de 4-diméthylaminopyridine. On obtient 6,8 g de produit attendu fondant à 260—262°C.

$/\alpha/_D = +66{,}5° \pm 2{,}5°$ (c=0,7% $CH_3COOH$).

*Analyse*

| | | | | | |
|---|---|---|---|---|---|
| Calculé: | C% 53,22 | H% 4,67 | N% 11,28 | F% 11,48 | S% 6,46 |
| Trouvé: | 52,9 | 4,8 | 11,1 | 11,5 | 6,4 |

Exemple 8

2-/(S) 1-aminopropyl/ 4-hydroxy N-(2-thiazolyl) 8-trifluoro-méthyl 3-quinoléine carboxamide.

On mélange, sous agitation, 6,5 g de produit de l'exemple 7, 130 cm3 de chlorure de méthylène et 65 cm3 d'acide trifluoroacétique, agite 2 heures à température ambiante, élimine le chlorure de méthylène et l'acide trifluoroacétique sous pression réduite. On reprend le résidu par de l'eau glacée et ajuste le pH à 7 avec une solution de carbonate acide de sodium, essore, lave, sèche sous pression réduite à 100°C. Le produit obtenu est dissous au reflux dans 120 cm3 d'acétonitrile, on filtre, concentre à 100 cm3, glace les cristaux obtenus, essore, lave, sèche sous pression réduite à 100°C. On obtient 3,25 g de produit attendu fondant à 206°C.

$/\alpha/_D = +177° \pm 2{,}5°$ (c=0,8% $CH_3COOH$).

*Analyse*

| | | | | | |
|---|---|---|---|---|---|
| Calculé: | C% 51,51 | H% 3,81 | N% 14,13 | F% 14,38 | S% 8,09 |
| Trouvé: | 51,5 | 3,8 | 14,0 | 14,5 | 8,0 |

Exemple 9

N-/(S)-1-/4-hydroxy 3-/(2-thiazolylamino) carbonyl/ 8-trifluorométhyl 2-quinoléinyl/ 2-méthylpropyl/ carbamate de 1,1-diméthy éthyle.

*Stade A:* N- /(S) (2-méthyl 1-(4-oxo 8-trifluorométhyl) 4H-3,1-benzoxazin-2-yl) propyl/ carbamate de 1,1-diméthyl éthyle.

On opère comme indiqué au stade A de l'exemple 7 à partir de 17,4 g de BOC—L-Valine en solution dans 200 cm3 de chlorure de méthylène, 20,2 cm3 de N-méthyl morpholine et de 20,8 cm3 de chloroformiate d'isobutyle dans 100 cm3 de chlorure de méthylène et de 16,4 g d'acide 2-amino 3 trifluorométhyl benzoïque en solution dans 200 cm3 de chlorure de méthylène et 8,8 cm3 de N-méthyl morpholine. On obtient 12 g de produit attendu fondant à 165°C.

$/\alpha/_D = -61° \pm 2{,}5°$ (c=0,6% $CH_3COOH$).

*Stade B:* N-/(S) 1-//2-/1,3-dioxo 3-(2-thiazolylamino) propyl/ 6-(trifluorométhyl) phényl/ aminocarbonyl/ 2-méthyl propyl/ carbamate de 1,1-diméthyl éthyle.

On opère comme indiqué au stade B de l'exemple 7 à partir de 13,25 g de 2-acétylaminothiazole dans 400 cm3 de tétrahydrofuranne, de 133 cm3 de n-butyllithium, de 12 g de produit obtenu au stade A précédent dans 85 cm3 de tétrahydrofuranne. On obtient 15,2 g de produit attendu fondant à 120°C.

$/\alpha/_D = -49{,}5° \pm 2°$ (c=0,9% $CH_3COOH$).

*Stade C:* N-/(S)-1-/4-hydroxy 3-/(2-thiazolylamino) carbonyl/ 8-trifluorométhyl 2-quinoléinyl/ 2-méthylpropyl/ carbamate de 1,1-diméthyl éthyle.

On opère comme indiqué au stade C de l'exemple 7 à partir de 15,2 g de produit obtenu au stade B précédent dans 150 cm3 de tétrahydrofuranne et de 4 g de 4-diméthylaminopyridine. On obtient 9,35 g de produit attendu fondant à 222°C.

$/\alpha/_D = +50{,}5° \pm 1°$ (c=1% $CH_3COOH$).

*Analyse*

| | | | | | |
|---|---|---|---|---|---|
| Calculé: | C% 54,11 | H% 4,94 | F% 11,16 | N% 10,97 | S% 6,28 |
| Trouvé: | 53,80 | 5,00 | 11,00 | 10,80 | 6,40 |

Exemple 10

Trifluoroacétate de 2-/(S) 1-amino 2-méthylpropyl/ 4-hydroxy N-(2-thiazolyl) 8-(trifluorométhyl) 3-quinoléine carboxamide.

On opère comme indiqué à l'exemple 8 à partir de 10,18 g de produit de l'exemple 9 dans 100 cm3 de chlorure de méthylène et de 50 cm3 d'acide trifluoroacétique. On obtient 10,2 g de produit attendu sous forme de trifluoroacétate fondant à 164°C.
/α/$_D$=−26,5°±1° (c=1,2% $CH_3COOH$).

*Analyse*

| | | | | | |
|---|---|---|---|---|---|
| Calculé: | C% 45,80 | H% 3,45 | F% 21,73 | N% 10,68 | S% 6,11 |
| Trouvé: | 45,60 | 3,50 | 21,80 | 10,70 | 6,10 |

Exemple 11

N-/(S)1-/4-hydroxy 3-/(2)-thiazolylamino) carbonyl/ 8-trifluoro-méthyl) 2-quinoléinyl/ 2-(1H-indol-3-yl) éthyl/ carbamate de 1,1-diméthyl éthyl.

*Stade A:* N-/(S) 2-/1H-indol-3-yl) 1-/4-oxo 8-trifluorométhyl) 4H-3,1-benzoxazin-2-yl/ éthyl/ carbamate de 1,1-diméthyl éthyle.

On opère comme indiqué au stade A de l'exemple 7 à partir de 22,8 g de BOC—L-tryptophane dans 150 cm3 de chlorure de méthylène et 20,6 g de N-méthyl morpholine, 19,5 cm3 de chloroformiate d'isobutyle dans 75 cm3 de chlorure de méthylène, 15,4 g d'acide 2-amino 3-triflurométhyl benzoïque dans 150 cm3 de chlorure de méthylène et 8,3 cm3 de N-méthyl morpholine. On obtient 20,7 g de produit attendu fondant à 166°C.
/α/$_D$=−52°±2,5° (c=0,4% $CH_3COOH$).

*Analyse*

| | | | | |
|---|---|---|---|---|
| Calculé: | C% 60,88 | H% 4,68 | N% 8,87 | F% 12,04 |
| Trouvé: | 61,2 | 4,8 | 8,8 | 11,9 |

*Stade B:* N-/(S) 1-//2-/1,3-dioxo 3-(2-thiazolylamino) propyl/ 6-trifluoro-méthyl/ phényl/ aminocarbonyl/ 2-(1H-indol-3-yl) éthyl/ carbamate de 1,1-diméthyl éthyle.

On opère comme indiqué au stade B de l'exemple 7 à partir de 18,34 g de 2 acétylaminothiazole dans 565 cm3 de tétrahydrofuranne, de 184 cm3 d'une solution de n-butyllithium dans l'hexane et de 20,3 g de produit obtenu au stade A précédent dans 150 cm3 de tétrahydrofuranne. On obtient 28 g d'une huile orange qui est utilisée telle quelle dans le stade suivant.

*Stade C:* N-/(S) 1-/4-hydroxy 3-/(2-thiazolylamino) carbonyl/ 8-trifluoro-méthyl) 2-quinoléinyl/ 2-(1H-indol-3-yl) éthyl/ carbamate de 1,1-diméthyl éthyle.

On opère comme indiqué au stade C de l'exemple 7 à partir de 28 g de produit obtenu au stade B précédent dans 280 cm3 de tétrahydrofuranne et de 5,25 g de diméthylaminopyridine. On obtient 16,9 g de produit attendu fondant à 270°C.
/α/$_D$=+33°±2° (c=0,5% $CH_3COOH$).

*Analyse*

| | | | | | |
|---|---|---|---|---|---|
| Calculé: | C% 58,28 | H% 4,38 | N% 11,72 | F% 9,54 | S% 5,36 |
| Trouvé: | 58,3 | 4,4 | 11,4 | 9,3 | 5,4 |

Exemple 12

2-/(S) 1-amino 2-(1H-indol-3-yl) éthyl/ 4-hydroxy N-(2-thiazolyl) 8-trifluorométhyl) 3-quinoléine carboxamide.

On mélange pendant 3 heures à température ambiante 16,9 g de produit obtenu a l'exemple 11, 170 cm3 de chlorure de méthylène et 85 cm3 d'acide trifluoroacétique. On élimine les solvants sous pression réduite, obtient une huile brute qui cristallise dans l'éther; on essore, rince et sèche sous pression réduite à 50°C. On obtient 16,4 g de produit attendu sous forme de trifluoroacétate fondant vers 190°C.

*Retour à la base*

On introduit les 16,4 g précédents dans 200 cm3 d'eau, extrait avec 200 cm3 d'acétate d'éthyle, lave à l'eau, sèche, concentre sous pression réduite et cristallise dans l'éther. On essore, sèche sous pression réduite, dissout le résidu obtenu dans 100 cm3 d'acétonitrile à température ambiante. On glace, essore les cristaux, sèche sous pression réduite à 90°C. On obtient 7,830 g de produit brut que l'on recristallise dans l'isopropanol. Après avoir chassé sous pression réduite l'isopropanol, on empâte le résidu obtenu dans l'acétonitrile. On essore, sèche sous pression réduite, reprend le résidu dans 20 cm3 d'acétate d'éthyle, essore, lave, sèche sous pression réduite à 100°C. On obtient 4,8 g de produit attendu fondant à 190°C.
/α/$_D$=+47°±1° (c=1% DMF).

*Analyse*

| | | | | | |
|---|---|---|---|---|---|
| Calculé: | C% 57,94 | H% 3,65 | N% 14,08 | F% 11,46 | S% 6,44 |
| Trouvé: | 57,7 | 3,9 | 13,0 | 10,6 | 6,2 |

Exemple 13

N-/(S) 1-/4-hydroxy 3-/(2-thiazolylaminocarbonyl) 8-trifluoro-méthyl) 2-quinoléinyl/ 2-(phényl) éthyl/ carbamate de 1,1-diméthyl éthyle.

*Stade A:* N-/(S) 2-phényl 1-/4-oxo 8-(trifluorométhyl) 4H-3,1-benzoxazin-2-yl/ éthyl/ carbamate de 1,1-diméthyl éthyle.

On opère comme indiqué au stade A de l'exemple 7 à partir de 19,9 g de BOC—L-phénylalanine dans 150 cm3 de chlorure de méthylène et 20,6 cm3 de N-méthyl morpholine, de 19,5 cm3 de chloroformiate d'isobutyle dans 75 cm3 de chlorure de méthylène et de 15,4 g d'acide 2-amino 3-trifluorométhyl benzoïque dans 150 cm3 de chlorure de méthylène et 8,3 cm3 de N-méthyl morpholine. On obtient 14,5 g de produit attendu fondant à 145°C.

$/\alpha/_D = -33,5° \pm 2°$ (c=0,5% $CH_3COOH$).

*Analyse*

| | | | | |
|---|---|---|---|---|
| Calculé: | C% 60,83 | H% 4,87 | N% 6,45 | F% 13,12 |
| Trouve: | 60,6 | 4,9 | 6,5 | 12,8 |

*Stade B:* N-/(S) 1-//2-/1,3-dioxo 3-(2-thiazolylamino) propyl/ 6-(trifluorométhyl) phényl/ aminocarbonyl/ 2-(phényl) éthyl/ carbamate de 1,1-diméthyl éthyle.

On opère comme indiqué au stade B de l'exemple 7 à partir de 9,09 g de 2-acétylamiothiazole dans 280 cm3 de tétrahydrofuranne, de 91 cm3 de n-butyllithium en solution dans l'hexane, de 13,9 g de produit obtenu au stade A précédent dans 100 cm3 de tétrahydrofuranne. On obtient 19 g de produit attendu.

*Stade C:* N-/(S) 1-/4-hydroxy 3-/(2-thiazolylaminocarbonyl) 8-trifluorométhyl) 2-quinoléinyl/ 2-(phényl) éthyl/ carbamate de 1,1-diméthyl éthyle.

On opère comme inidiquè au stade C de l'exemple 7 à partir de 18,7 g de produit obtenu au stade B précédent, de 190 cm3 de tétrahydrofuranne et de 3,9 g de 4-diméthylaminopyridine. On obtient 17,4 g de produit attendu fondant à 268°C.

$/\alpha/_D = +44° \pm 3°$ (c=0,25% DMF).

*Analyse*

| | | | | | |
|---|---|---|---|---|---|
| Calculé: | C% 58,06 | H% 4,51 | N% 10,03 | F% 10,2 | S% 5,74 |
| Trouve: | 58,0 | 4,5 | 9,9 | 10,4 | 6,0 |

Exemple 14

2-/(S) 1-amino 2-phényléthyl/ 4-hydroxy N-(2-thiazolyl) 8-(trifluorométhyl) 3-quinoléine carboxamde.

On mélange 16,9 g de produit obtenu à l'exemple 13, 300 cm3 de chlorure de méthylène et 85 cm3 d'acide trifluoroacétique, agite 2 heures à température ambiante, élimine les solvants sous pression réduite. On obtient une huile qui cristallise spontanément dans l'éther. On essore, lave, sèche sous pression réduite et obtient 11,9 g de produit attendu sous forme de trifluoroacétate fondant à 190°C.

*Retour à la base*

On introduit le produit obtenu ci-dessus dans 100 cm3 d'eau, agite, ajoute une solution aqueuse saturée de carbonate acide de sodium jusqu'à l'obtention de pH 8, agite 30 minutes à température ambiante. On essore, lave à l'eau plusieurs fois jusqu'à obtention d'un pH de 2—3, sèche sous pression réduite et obtient 9,3 g de produit brut.

*Recristallisation*

On dissout le produit obtenu ci-dessus dans 80 cm3 de diméthyl formamide et filtre; on additionne 160 cm3 d'éther au filtrat. On glace, essore les cristaux obtenus, lave à l'éther, sèche sous pression réduite à température ambiante et obtient 2,5 g de produit attendu fondant à 248—250°C. (lère forme cristalline). On obtient un 2ème jet en reprenant le filtrat diméthylformamide-éther précédent. On élimine sous pression réduite les solvants et obtient une huile orange qui cristallise dans l'éther. On essore, lave sèche sous pression réduite, dissout le résidu dans du tétrahydrofuranne, filtre, concentre le filtrat et ajoute de l'éther. On glace, essore, lave, sèche sous pression réduite à 90°C les cristaux obtenus et obtient 4,52 g de produit attendu fondant à 234°C (2ème forme cristalline).

Exemple 15

(2S)-2-/4-hydroxy 3-/(2-thiazolylamino) carbonyl/ 8-trifluorométhyl 2-quinoléinyl/ 1-pyrrolidine carboxylate de 1,1-diméthyl éthyle.

*Stade A:* (2S) 2-/4-oxo 8-(trifluorométhyl) 4H-3,1-benzoxazin-2-yl/ 1-pyrrolidine carboxylate de 1,1-diméthyléthyle.

On opère comme au stade A de l'exemple 7 à partir de 16,14 de BOC—L-proline en solution dans 150 cm3 de chlorure de méthylène, 20,6 cm3 de N-méthyl morpholine, de 19,5 cm3 de chloroformiate d'isobutyle dans 75 cm3 de chlorure de méthylène et de 15,4 g d'acide 2-amino 3-trifluorométhyl benzoïque dans 150 cm3 de chlorure de méthylène et 8,3 cm3 de N-méthyle morpholine. Après 20 heures de réaction, on extrait au chlorure de méthylène, chromatographie sur silice (éluant: chlorure de mèthylène) et obtient 16,4 g de produit attendu.

$/\alpha/_D = -75° \pm 2.5°$ (c=0,6% dans $CH_3COOH$).

*Stade B:* (2S) 2-//2-/1,3-dioxo 3-(2-thiazolylamino) propyl/ 6-trifluorométhyl phényl/ aminocarbonyl/ 1-pyrrolidine carboxylate de 1,1-diméthyléthyle.

On opère comme au stade B de l'exemple 1 à partir de 11,8 g de N-2-thiazolyl acétamide dans 360 cm3 de tétrahydrofuranne, de 119 cm3 d'une solution de N-butyllithium dans l'hexane et de 16 g de produit obtenu au stade A précédent dans 100 cm3 de tétrahydrofuranne. On obtient 9,0 g de produit attendu. F=136—138°c.

$/\alpha/_D = -49° \pm 1°$ (c=1% $CH_3COOH$).

y*Analyse:* $C_{23}H_{25}N_4F_3SO_5$: 526,543).

| | | | | | |
|---|---|---|---|---|---|
| Calculé: | C% 52,47 | H% 4,78 | N% 10,64 | F% 10,83 | S% 6,09 |
| Trouve: | 52,8 | 4,9 | 10,3 | 10,7 | 6,0 |

*Stade C:* (2S) 2-/4-hydroxy 3-/(2-thiazolylamino) carbonyl/ 8-trifluorométhyl-2-quinoléinyl/ 1-pyrrolidine carboxalate de 1,1-diméthyl éthyle.

On agite 24 heures à température ambiante 11,1 g de produit obtenu au stade B précédent, 110 cm3 de tétrahydrofuranne et 2,6 g de 4-diméthyl aminopyridine. On porte au reflux, élimine le tétrahydrufuranne sous pression réduite, reprend le résidu par 100 cm3 d'eau et 10,5 cm3 d'acide chlorhydrique 2N, et dissout le précipité dans l'acétate d'éthyle. On lave la solution organique à l'eau, la sèche, la concentre à sec sous pression réduite et obtient après recristallisation dans l'éther, 9,1 g de produit attendu. F=215°C.

$/\alpha/_D = -55° \pm 1,5°$ (c=1% $CH_3COOH$).

*Analyse:* $C_{23}H_{23}N_4O_4F_3S$: 508,538

| | | | | | |
|---|---|---|---|---|---|
| Calculé: | C% 54,32 | H% 4,56 | N% 11,02 | F% 11,21 | S% 6,30 |
| Trouvé: | 54,5 | 4,7 | 10,8 | 11,1 | 6,2 |

## Exemple 16

4-hydroxy 2-/(2S) 2-pyrrolidinyl/ N-(2-thiazolyl) 8-trifluorométhyl 3-quinoléine carboxamide.

On opère comme à l'exemple 14 à partir de 7,8 g de produit obtnu à l'exemple 11, 80 cm3 de chlorure de méthylène, 40 cm3 d'acide trifluoroacétique et obtient 8,0 g de produit attendu sous forme de trifluoroacétate. F≃ 210°C.

Retour à la base:

On introudit les 8 g de trifluoracétate dans 100 cm3 d'eau, extrait à l'acétate d'éthyle, lave à l'eau jusqu'à pH=4, sèche et concentre sous pression réduite. On reprend le résidu par 80 cm3 d'acétonitrile, essore les cristaux obtenus et le sèche à 80°C sous pression réduite. On obtient 4,7 g de produit attendu. F=196°C.

$/\alpha/_D = -7° \pm 1°$ (c=1% $CH_3COOH$).

*Analyse:* $C_{18}H_{15}F_3N_4O_2S$: 408,411

| | | | | | |
|---|---|---|---|---|---|
| Calculé: | C% 52,94 | H% 3,70 | N% 13,72 | F% 13,96 | S% 7,85 |
| Trouve: | 52,4 | 3,6 | 13,4 | 13,9 | 7,8 |

## Exemple 17

N-/1-/4-hydroxy 3-/(2-thiazolylamino) carbonyl/ 8-trifluorométhyl 2-quinoléinyl/ 2-(phénylméthoxy) éthyl/ carbamate de 1,1-diméthyléthyle.

*Stade A:* N-/1-/4-oxo 8-trifluorométhyl 4H-3,1-benzoxazin-2-yl/ 2-(phénylmethoxy) éthyl/ carbamate de 1,1-diméthyléthyle.

On opère comme au stade A de l'exemple 7 à partir de 29,5 g de BOC—O-benzyl L-serine dans 200 cm3 de chlorure de méthylène, 27,5 cm3 de N-méthyl morpholine, de 26,2 g de chloroformiate d'isobutyle dans du chlorure de méthylène, et de 20,5 g d'acide 2-amino 3-trifluorométhyl benzoïque dans 200 cm3 de chlorure de méthylène et 11 cm3 de N-méthyl morpholine. Après 16 heures de réaction, on extrait au chlorure de méthylène, chromatographie sur silice (éluant: chlorure de méthylène) et obtient 18,5 g de produit attendu. F=98°C.

*Analyse:* $C_{23}H_{23}N_2O_5F_3$: 464,449

| | | | | |
|---|---|---|---|---|
| Calculé: | C% 59,48 | H% 4,49 | N% 6,03 | F% 12,27 |
| Trouve: | 59,2 | 5,0 | 6,0 | 12,3 |

*Stade B:* N-/2-/2-/1,3-dioxo 3-/(2-thiazolyl) amino/ propyl/ 6-trifluorométhylphényl/ amino/ 2-oxo 1-/ (phénylméthoxy) méthyl/ éthyl/ carbamate de 1,1-diméthyléthyle.

On opère comme au stade B de l'exemple 1 à partir de 11,14 g de N-2-thiazolyl acétamide dans 350 cm3 de tétrahydrofuranne, de 112 cm3 de N-butyllithium dans l'hexane et de 18,2 g de produit obtenu au stade A précédent dans 100 cm3 de tétrahydrofuranne. On obtient 15,8 g de produit attendu. F=134°—136°C.

*Anaylse:* $C_{22}H_{29}N_4O_6F_3S$: 606,63

| | | | | | |
|---|---|---|---|---|---|
| Calculé: | C% 55,54 | H% 4,82 | N% 9,24 | F% 9,40 | S% 5,28 |
| Trouve: | 55,7 | 4,7 | 9,3 | 9,4 | 5,4 |

*Stade C:* N-/1-/4-hydroxy 3-/(2-thiazolylamino) carbonyl/ 8-trifluorométhyl 2-quinoléinyl/ 2-(phénylméthoxy) éthyl/ carbamate de 1,1-diméthyléthyle.

On agite 6 heures à température ambiante 15,5 g de produit obtenu au stade B précédent dans 150 cm3 de tétrahydrofuranne et 3,1 g de 4-diméthylaminopyridine. On verse la solution dans 400 cm3 d'eau additionnée de 20 cm3 d'acide chlorhydrique 2N, extrait avec un mélange d'acétate d'éthyle et de tétrahydrofuranne. On lave la phase organique à l'eau, sèche, concentre à sec sous pression réduite, cristallise le résidu dans l'éther et obtient après séchage à 70°C sous pression réduite 13,6 g de produit attendu. F=208°C.

*Anaylse:* $C_{28}H_{27}O_5N_4F_3S$: 588,615

| | | | | | |
|---|---|---|---|---|---|
| Calculé: | C% 57,14 | H% 4,62 | N% 9,52 | F% 9,68 | S% 5,44 |
| Trouve: | 57,1 | 4,5 | 9,5 | 9,9 | 5,5 |

## Exemple 18

2-(1-amino 2-hydroxyéthyl) 4-hydroxy N-(2-thiazolyl) 8-trifluorométhyl 3-quinoléine carboxamide.

On refroidit à −20°C, 10,3 g de produit obtenu à l'exemple 17 dans 300 cm3 de chlorure de méthylène et ajoute 131,25 cm3 d'une solution molaire de tribromure de bore dans le chlorure de méthylène. On maintient l'agitation 1 heure à −20°C puis 2 heures à température ambiante. On verse le mélange dans 800 cm3 d'eau, extrait avec du chlorure de méthylène, de l'acétate d'éthyle et du tétrahydrofuranne, lave 1a phase organique à l'eau, la sèche et la concentre à sec sous pression réduite. Après cristallisation dans l'éther, on obtient en 2 jets, 4,9 g de produit brut que l'on dissout dans 180 cm3 de tétrahydrofuranne et 50 cm3 d'eau, ajoute du charbon actif, filtre sur silice, concentre sous pression réduite jusqu'à l'apparition de cristaux. On glace, essore le produit cristallisé, le lave au tétrahydrofuranne, le sèche à 80°C sous pression réduite et obtient 2,7 g de produit attendu. F=248°C.

*Analyse:* $C_{16}H_{13}O_3N_4F_3S$: 398,371

| | | | | | |
|---|---|---|---|---|---|
| Calculé: | C% 48,24 | H% 3,29 | N% 14,06 | F% 14,31 | S% 8,05 |
| Trouve: | 47,9 | 3,3 | 13,6 | 14,4 | 8,0 |

## Exemple 19

2-/(1S) 1-(acétylamino) propyl/ 4-hydroxy N-(2-thiazolyl) 8-trifluorométhyl 3-quinoléine carboxamide.

On ajoute goutte à goutte sous agitation 10 cm3 d'anhydride acétique, 6 g de 2-/(S) 1-aminopropyl/ 4-hydroxy N-(2-thiazolyl) 8-trifluorométhyl 3-quinoléine carboxamide obtenu comme à l'exemple 8 dans 60 cm3 de pyridine et maintient l'agitation 1 heure 30 à température ambiante. On verse la solution sur 150 cm3 d'eau glacée, ajoute de l'acide chlorhydrique concentré jusqu'à pH=1. On essore le précipité, le lave à l'eau, le dissout dans le tétrahydrofuranne. On sèche la phase organique, la concentre sous pression réduite, reprend le résidu à l'éther, l'essore et le sèche sous pression réduite à 80°C. On recueille 5,3 g de produit attendu. F=275°C.

$/\alpha/_D = +43{,}5° \pm 4°$ (c=0,3% DMF).

*Analyse:* $C_{19}H_{17}O_3N_4F_3S$: 438,436

| | | | | | |
|---|---|---|---|---|---|
| Calculé: | C% 52,05 | H% 3,91 | N% 12,78 | F% 13,00 | S% 7,31 |
| Trouve: | 51,8 | 3,8 | 12,4 | 13,1 | 7,2 |

## Exemple 20

4-hydroxy 2-/(S) 1-(1-oxopropyl) amino/ propyl/ N-(2-thiazolyl) 8-trifluorométhyl 3-quinoléine carboxamide.

On opère comme à l'exemple 19 au départ de 3,2 g de 2-/(S) 1-amino propyl/ 4-hydroxy N-(2-thiazolyl) 8-trifluorométhyl/ 3-quinoléine carboxamide préparé comme à l'exemple 8, de 32 cm3 de pyridine et de 6,6 cm3 d'anhydride propionique. On obtient 3,0 g de produit attendu. F>275°C.

$/\alpha/_D = +57{,}5°C + 2{,}5°$ (c=0,5% $CH_3COOH$).

*Analyse:* $C_{20}H_{19}F_3N_4O_3S$: 452,458

| | | | | | |
|---|---|---|---|---|---|
| Calculé: | C% 53,09 | H% 4,23 | N% 12,38 | F% 12,60 | S% 7,09 |
| Trouve: | 53,0 | 4,3 | 12.1 | 12,8 | 7,1 |

Exemple 21

N-(S)-/2-//4-hydroxy 3-/(2-thiazolylamino) carbonyl/ 8-trifluorométhyl 2-quinoléinyl/ 2-méthylpropyl/ amino/ 2-oxoéthyl/ carbamate de 1,1-diméthyléthyle.

On refroidit à −25°C la solution comprenant 4,38 g de N—BOC-glycine, 40 cm3 de chlorure de méthylène et 2,75 cm3 de N-méthyl morpholine et ajoute 3,41 cm3 de chloroformiate d'isobutyle en soution dans 30 cm3 de chlorure de méthylène. On agite 25 minutes à −25°C, ajoute 8,56 g de 2-/(S) 1-aminó 2-méthylpropyl/ 4-hydoxy N-(2-thiazolyl) 8-trifluorométhyl 3-quinoléine carboxamide prépare selon un mode opératoire identique à celui de l'exemple 12 à partir de trifluoroacétate obtenu comme à l'exemple 8, en solution dans 80 cm3 de tétrahydrofuranne et maintient l'agitation 1 heure à −25°C. On verse le mélange réactionnel sur 300 cm3 d'eau additionnée d'acide chlorhydrique 2N jusqu'à l'obtention d'un pH=1. On extrait au chlorure de méthylène, lave la phase organique à l'eau, puis avec une solution aqueuse saturée de carbonate acide de sodium, puis à l'eau, sèche et concentre à sec sous pression réduite. On reprend le résidue huileux dans l'éther de pétrole (Eb=60°C—80°C), essore les cristaux obtenus, les sèche à 60°C sous pression réduite et obtient 11,15 g de produit attendu.l F≃170°C.

$/\alpha/_D=+37°+2°$ (c=0,5% $CH_3COOH$).

Exemple 22

2-/(1S) 1-/(aminoacétyl) amino/ 2-méthylpropyl/ 4-hydroxy N-(2-thiazolyl) 8-trifluorométhyl 3-quinoléine carboxamide (monohydrate).

Dans une solution comprenant 9,6 g de produit obtenu à l'exemple 21 dans 50 cm3 de chlorure de méthylène, on ajoute goutte à goutte sous agitation 20 cm3 de solution éthanolique d'acide chlorhydrique (5,7N), agite 16 heures à température ambiante, ajoute de nouveau 10 cm3 de solution éthanolique et agite 1 heure. On élimine les solvants sous pression réduite à une température inférieure à 40°C, dissout le résidu dans l'eau, ajoute du charbon actif et filtre sur silice. On ajuste le pH à 8 avec une solution aqueuse saturée de carbonate acide de sodium, essore, lave à l'eau et sèche pression réduite à 70°C. On obtient 6,7 g de produit brut. On dissout 4,48 g de ce produit dans 20 cm3 de diméthylformamide à température ambiante, filtre sur silice, ajoute 25 cm3 d'eau, glace, essore et sèche sous pression réduite à 50°C pendant 16 heures le produit cristallisé. On obtient 3,6 g de produit attendu. F=230°C.

$/\alpha/_D=+72\pm1{,}5°$ (c=1% $CH_3COOH$).

*Analyse:* $C_{20}H_{20}N_5O_3F_3S$, $H_2O$: 485,50

| | | | | | |
|---|---|---|---|---|---|
| Calculé: | C% 49,47 | H% 4,56 | N% 14,42 | F% 11,74 | S% 6,60 |
| Trouve: | 49,1 | 4,7 | 14,3 | 11,3 | 6,4 |

Exemples de compositions pharmaceutiques

Exemple 23

On a préparé des comprimés répondant à la formule suivante:

| | |
|---|---|
| Produit de l'exemple 6 | 50 mg |
| Excipient q.s. pour un comprimé terminé à | 350 mg |

(détail de l'excipient: lactose, talc, amidon, stéarate de magnésium).

Exemple 24

On a préparé des comprimés répondant à la formule suivante:

| | |
|---|---|
| Produit de l'exemple 8 | 30 mg |
| Excipient q.s. pour un comprimé terminé à | 350 mg |

(détail de l'excipient: lactose, talc, amidon, stéarate de magnésium).

Etude Pharmacologique des Produits des Exemples 6 et 8

1) Etude de l'activité analgésique.

Le test employé est basé sur le fait signalé par R. KOSTER et Cool. (Fed. Proc. 1959, *1B* 412) selon lequel l'injection intrapéritonéale d'acide acétique provoque, chez la souris, des mouvements répétés d'étirement de torsion pouvant persiste pendant plus de 6 heures. Les analgésiques préviennent ou diminuent ce syndrome qui peut être considéré comme l'extériorisation d'une douleur abdominale diffuse. On utilise une solution d'acide acétique à 1% dans l'eau. La dose déclenchant le syndrome est dans ces conditions de 0,01 cm3/g, soit 100 mg/kg d'acide acétique.

Le produit étudié est administré par voie intra-veineuse une demiheure avant l'injection d'acide acétique, les souris étant à jeun depuis la veille de l'expérience.

Les étirements sont observés et comptés pour chaque souris pendant une période d'observation de 15 minutes commençant aussitôt après l'injection acétique.

Les résultats sont exprimés au moyen de la $DA_{50}$, c'est-à-dire la dose qui permet d'obtenir une diminution de 50% du nombre des étirements par rapport aux animaux témoins. La $DA_{50}$ trouvée a été de 2 mg/kg et 7 mg/kg respectivement pour les produits des exemples 6 et 8.

2) Activité anti-inflammatoire: arthrite chronique de l'adjuvant (traitement préventif).

L'injection d'adjuvant de type "Freund" dans une patte postérieure provoque, chez le rat, l'apparition rapide d'une lésion inflammatoire primaire dans cette patte, puis, après un temps de latence de 13 à 15 jours, le déclenchement d'une arthrite secondaire affectant notamment l'autre pate postérieure. Le test est pratiqué sur des rats mâles âgés de 42 à 50 jours, qui reçoivent en injection intraplantaire, 0,1 ml d'adjuvant de type "Freund" (suspension dans l'huile de vaseline de 6 mg par ml de mycobacterium butyricum tués).

Les animaux reçoivent le produit étudié, par voie orale, du jour 0 (jour de l'injection de l'adjuvant) jusqu'à la veille du sacrifice, pratiqué le jour 17. Des animaux témoins arthritiques, des animaux témoins normaux ne reçoivent que le véhicule. Les critères d'appréciation de l'activité des substances étudiées sont les augmentations de volume des pattes postérieures injectées (inflammation primaire et secondaire) et non injectées (inflammation secondaire) par rapport au volume moyen des pattes correspondantes de témoins normaux.

On détermine la $DA_{50}$, c'est-à-dire la dose qui diminue de 50% les augmentations de volume des pattes postérieures des animaux traités par rapport aux animaux témoins.

La $DA_{50}$ trouvée été de 5 mg/kg et de 3 mg/kg respectivement pour les produits des exemples 6 et 8.

**Revendications**

1. Les composés de formule (I):

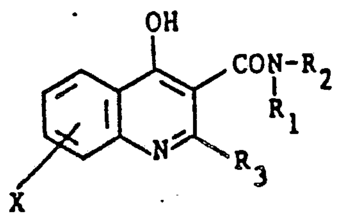

(I)

dans laquelle X en position 5, 6, 7 ou 8 représente un atome d'hydrogène, un atome d'halogène, un radical alcoyle linéaire ou ramifié renfermant de 1 à 5 atomes de carbone, un radical alcoxy linéaire our ramifié renfermant de a à 4 atomes de carbone, un radical trifluorométhyle, un radical trifluoromethylthio ou un radical trifluorométhoxy, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle et tétrazoyle, éventuellement substitués par un radical alcoyle renfermant de 1 à 4 atomes de carbone et phényle, éventuellement substitué par un ou plusieurs radicaux choisis dans le groupe formé par le radical hydroxy, les radicaux alcoyles renfermant de 1 à atomes de carbone, les radicaux alcoxy renfermant de 1 à atomes de carbone, le radical trifluorométhyle, le radical nitro et les atomes d'halogène, $R_3$ représente un radical 2-pyrrolidinyle de formule

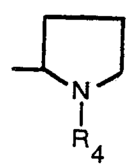

ou un radical

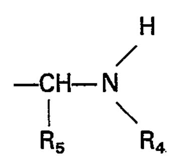

dans lesquels $R_4$ représente soit un atome d'hydrogène, soit un groupe

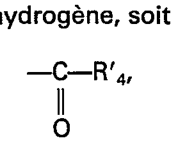

$R'_4$ étant un radical alcoyle renfermant de 1 à 5 atomes de carbone éventuellement substitué par un radical amino libre ou protégé, un radical phényle ou benzyle, soit un radical protecteur de la fonction amino, $R_5$ représente un atome d'hydrogène un radical alcoyle renfermant de 1 à 5 atomes de carbone, un radical phényle ou benzyle, un radical 4—OH benzyle, un radical 1H-indol 3-yl méthyl, de formule:

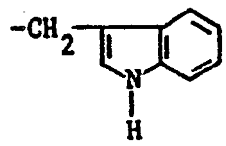

un radical

$$-CH_2OH \text{ ou } -\underset{OH}{\underset{|}{CH}}-CH_3 \text{ ou } -CH_2SH,$$

les fonctions alcool et thiol de ces trois derniers radicaux étant éventuellement bloquées par des groupes protecteurs, sous leurs formes racémiques ou optiquement actives, ainsi que leurs sels d'addition avec les acides.

2. Les composés de formule (I) tels que définis à la revendication 1, pour lesquels X est en position 8 et ceux pour lesquels X représente un radical trifluorométhyle, sous leurs formes racémiques ou optiquement actives, ainsi que leurs sels d'addition avec les acides.

3. Les composés de formule (I) tels que définis à l'une des revendications 1 ou 2, pour lesquels $R_1$ représente un atome d'hydrogène, et pour lesquels $R_2$ représente le radical thiazolyle, sous leurs formes racémiques ou optiquement actives, ainsi que leurs sels d'addition avec les acides.

4. Les composés de formule (I), tels que définis aux revendications 1 à 3, pour lesquels les radicaux protecteurs de la fonction amino sont des groupements de formule $-CO_2A$, A étant un radical alcoyle renfermant de 1 à 5 atomes de carbone, un radical aryle ou arylalcoyle, sous leurs formes racémiques ou optiquement actives, ainsi que leurs sels d'addition avec les acides.

5. Les composés de formule (I), tels que définis aux revendications 1 à 4, pour lesquels A est un radical terbutyle ou benzyle, sous leurs formes racémiques ou optiquement actives, ainsi que leurs sels d'addition avec les acides.

6. Les composés de formule (I), tels que définis à l'une quelconque des revendications 1 à 3, pour lesquels $R_4$ représente un atome d'hydrogène, sous leurs formes racémiques ou optiquement actives, ainsi que leurs sels d'addition avec les acides.

7. Les composés de formule (I), tels que définis à l'une quelconque des revendications 1 à 6, sous leur forme optiquement active (S), ainsi que leurs sels d'addition avec les acides.

8. Procédé de préparation des produits de formule (I), dans laquelle X, $R_1$, $R_2$ et $R_3$ ont les significations données précédemment, caractérisé en ce que l'on soumet un composé de formule (II):

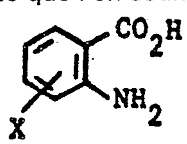

(II)

dans laquelle X conserve la signification donnée ci-dessus, à l'action d'un acide aminé de formule (III):

$$R_3-COOH \qquad (III)$$

sous forme activée et sous forme racémique ou optiquement active, dans lequel $R_3$ est un groupe:

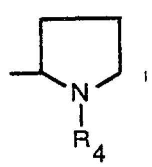

ou un groupe

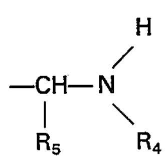

$R_4$ étant soit un groupe

$$-\underset{O}{\underset{\|}{C}}-R'_4,$$

$R'_4$ ayant la signification donnée précédemment, soit un radical protecteur de la fonction amino, $R_5$ ayant la signification donnée précédemment, étant entendu que lorsque $R_5$ représente un groupe

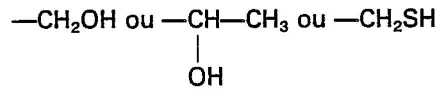

et lorsque $R'_4$ représente un radical alcoyle, substitué par un radical amino, les fonctions alcool, thiol et amine, sont éventuellement bloquées, pour obtenir un composé formule (IV), sous forme racémique ou optiquement active:

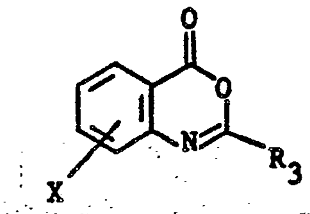

(IV)

dans laquelle X et $R_3$ conservent leurs significations précédentes, que l'on soumet, en présence d'une base, à l'action d'un composé de formule (V):

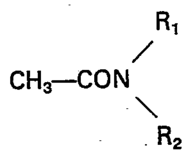

(V)

dans laquelle $R_1$ et $R_2$ conservent leurs significations précédentes, pour obtenir un composé de formule (VI) sous forme racémique ou optiquement active

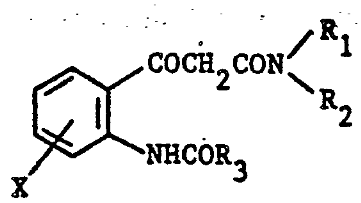

(VI)

dans laquelle X, $R_1$, $R_2$ et $R_3$ conservent leurs significations précédentes, que l'on cyclise en présence d'un agent alcalin, pour obtenir un composé de formule (I) dans laquelle X, $R_1$, $R_2$ et $R_3$ ont les significations qui viennent d'être données sous forme racémique ou optiquement active, que l'on soumet, le cas échéant, à l'action d'un ou plusieurs agents de déblocage des fonctions amino, alcool et thiol, pour obtenir un produit de formule (I) dans laquelle X, $R_1$ et $R_2$ ont les significations précédentes et dans laquelle $R_3$ représente soit un groupe:

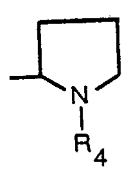

soit un groupe

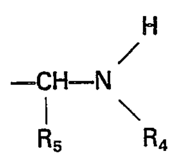

dans lesquels $R_4$ est un groupe

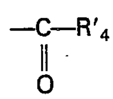

ou un atome d'hydrogène et $R_5$ a la signification précédente, étant entendu que lorsque $R_5$ représente un groupement

$$CH_2OH,\ \underset{\displaystyle OH}{\underset{|}{CH}}-CH_3m,\ CH_2SH$$

et lorsques $R'_4$ représente un radical alcoyle substitué par un radical amino, les fonctions alcool, thiol et amine sont libres, produits de formule (I), sous forme racémique ou optiquement active que l'on soumet, si désiré, à l'action d'un acide pour en former le sel.

9. Procédé de préparation des produits de formule (I), telle que définié à la revendication 1, dans laquelle X, $R_1$ et $R_2$ ont les significations données précédemment et $R_3$ représente un radical

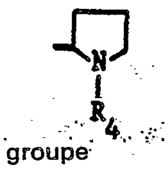 ou 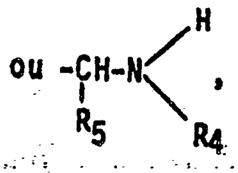 ,

dans lequel $R_4$ représente un groupe

$$-\overset{\displaystyle O}{\overset{\|}{C}}-R'_4$$

$R'_4$ étant un radical alcoyle renfermant de 1 à 5 atomes de carbone, éventuellement substitue par un radical amino libre ou protégé, un radical phenyl ou benzyle, caractérisé en ce que l'on fait réagir un produit de formule (I) dans laquelle $R_3$ represente un radical 2-pyrrolidinyle ou un radical

$$-\underset{\displaystyle R_5}{\underset{|}{CH}}-NH_2,$$

sous forme racémique ou optiquement active, avec un dérivé réactif du groupement

$$-\overset{\displaystyle O}{\overset{\|}{C}}-R'_4,$$

pour obtenir un produit de formule (I) correspondant, sous forme racémique ou optiquement active, que l'on soumet, le cas échéant, à l'action d'un agent de déblocage de la fonction amino, produit de formule (I), que l'on soumet, si désiré, à l'action d'un acide pour en former le sel.

10. A titre de médicaments, les produits tels que définis à l'une quelconque des revendications 1 à 5, sous leurs formes racémiques ou optiquement actives, ainsi que leurs sels d'addition avec les acides pharmaceutiquement acceptables.

11. A titre de médicaments, les produits tels que définis à la revendication 6, sous leurs formes racémiques ou optiquement actives, ainsi que leurs sels d'addition avec les acides pharmaceutiquement acceptables.

12. A titre de médicaments, les produits tels que définis à la revendication 7, ainsi que leurs sels d'addition avec les acides pharmaceutiquement acceptables.

13. Les compositions pharmaceutiques contenant à titre de principe actif, l'un au moins des médicaments, tels que définis aux revendications 10 à 12.

14. A titre de produits industrielles nouveaux, notamment à titre de produits intermédiaires, les produits de formule (VI), telle que definie à la revendication 8.

**Claims**

1. The compounds of formula (I):

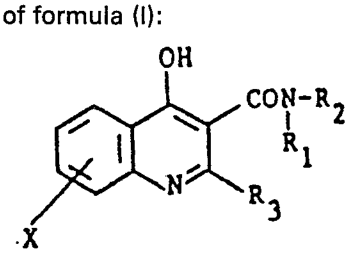

(I)

in which X at position 5, 6, 7 or 8 represents a hydrogen atom, a halogen atom, a linear or branched alkyl radical containing from 1 to 5 carbon atoms, a linear or branched alkoxy radical containing from 1 to 4 carbon atoms, a trifluoromethyl radical, trifluoromethylthio radical or a trifluoromethoxy radical, $R_1$ represents a hydrogen atom or an alkyl radical containing from 1 to 4 carbon atoms, $R_2$ represents a radical chosen from the following radicals, thiazolyl, 4,5-dihydrothiazolyl, pyridinyl, oxazolyl, isoxazolyl, imidazolyl, pyrimidyl and tetrazolyl, optionally substituted by an alkyl radical containing from 1 to 4 carbon atoms and phenyl, optionally substituted by one or more radicals chosen from the following group; hydroxy radical, alkoxy radicals containing from 1 to 4 carbon atoms, the trifluoromethyl radical, the nitro radical and halogen atoms, $R_3$ represents a 2-pyrrolidinyl radical of formula

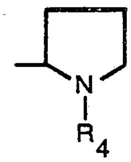

or a 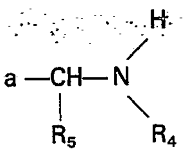 radical

in which $R_4$ represents either a hydrogen atom, or a

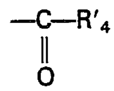 group,

$R'_4$ being an alkyl radical containing from 1 to 5 carbon atoms optionally substituted by a free or protected amino radical, a phenyl or benzyl radical, or a protector radical for the amino function, $R_5$ represents a hydrogen atom, an alkyl radical containing from 1 to 5 carbon atoms, a phenyl or benzyl radical, a 4-OH benzyl radical, a 1H-indol-3-yl methyl radical of formula:

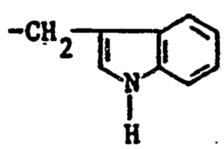

a $—CH_2OH$ or 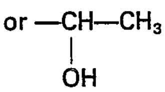 or $—CH_2SH$ radical,

the alcohol and thiol functions of these last three radicals being optionally blocked by protector groups, in their racemic or optionally active forms, as well as their addition salts with acids.

2. The compounds of formula (I) as defined in claim 1, in which X is at position 8 and those in which X represents a trifluoromethyl radical, in their racemic or optically active forms, as well as their addition salts with acids.

3. The compounds of formula (I) as defined in one of claims 1 or 2, in which $R_1$ represents a hydrogen atom, and in which $R_2$ represents a thiazolyl radical, in their racemic or optically active forms, as well as their addition salts with acids.

4. The compounds of formula (I), as defined in claims 1 to 3, in which the protector radicals of the amino function are groups of formula $—CO_2A$, A being an alkyl radical containing from 1 to 5 carbon atoms, an aryl or arylalkyl radical, in their racemic or optically active forms, as well as their addition salts with acids.

5. The compounds of formula (I), as defined in claims 1 to 4, in which A is a terbutyl or benzyl radical, in their racemic or optically active forms, as well as their addition salts with acids.

6. The compounds of formula (I), as defined in any one of claims 1 to 3, in which $R_4$ represents a hydrogen atom, in their racemic or optically active forms, as well as their addition salts with acids.

7. The compounds of formula (I), as defined in any one of claims 1 to 6, in their optically active form (S), as well as their addition salts with acids.

8. Preparation process for products of formula (I), in which X, $R_1$, $R_2$, and $R_3$ have the meanings given previously, characterised in that a compound of formula (II);

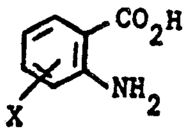

(II)

in which X retains the meaning given above, is subjected to the action of an aminated acid of formula (III):

$$R_3—COOH \qquad (III)$$

in the activated form and in the racemic or optically active form, in which $R_3$ is the following group:

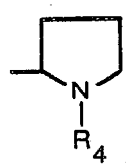

or a $—\underset{R_5}{\underset{|}{CH}}—N\begin{smallmatrix} H \\ R_4 \end{smallmatrix}$ group

$R_4$ being either a

$$—\underset{O}{\underset{\|}{C}}—R'_4 \text{ group,}$$

$R'_4$ having the meaning given previously, or a protector radical for the amino function, $R_5$ having the meaning given previously, it being understood that when $R_5$ represents a

$$—CH_2OH \text{ or } —\underset{OH}{\underset{|}{CH}}—CH_3 \text{ or } —CH_2SH \text{ group}$$

and when $R'_4$ represents an alkyl radical, substituted by an amino radical, the alcohol, thiol and amine functions are optionally blocked, so as to obtain a compound of formula (IV), in the racemic, or optically active form:

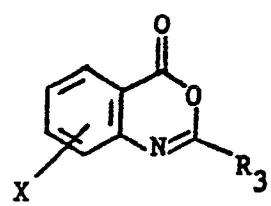

(IV)

in which X and $R_3$ retain their previous meanings, which is subjected, in the presence of a base, to the action of a compound of formula (V):

$$CH_3—CON\begin{smallmatrix} R_1 \\ R_2 \end{smallmatrix} \qquad (V)$$

in which $R_1$ and $R_2$ retain their previous meanings, so as to obtain a compound of formula (VI) in the racemic or optically active form:

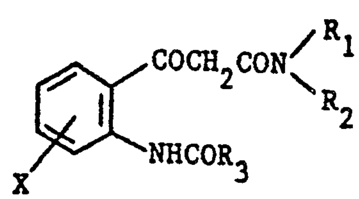

(VI)

in which X, $R_1$, $R_2$ and $R_3$ retain their previous meanings, which is cyclised in the presence of an alkaline agent, so as to obtain a compound of formula I in which X, $R_1$, $R_2$, and $R_3$ have the meanings that have just been given in the racemic or optically active form, which if the case arises, is subjected to the action of one or more deblocking agents of the amino, alcohol and thiol functions, so as to obtain a product of formula (I) in which X, $R_1$ and $R_2$ have the previous meanings and in which $R_3$ represents either the following group:

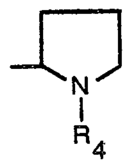

or a 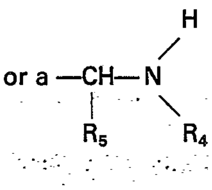 group

in which $R_4$ is a

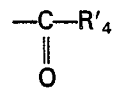

group or a hydrogen atom and $R_5$ has the previous meaning, it being understood that when $R_5$ represents a

$CH_2OH$, 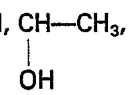, $CH_2SH$ group

and when $R'_4$ represents an alkyl radical substituted by an amino radical, the alcohol, thiol and amino functions are free, which products of formula (I), in the racemic or optically active form, are subjected, if desired, to the action of an acid to form the salt.

9. Preparation process for products of formula (I), as defined in claim 1, in which X, $R_1$ and $R_2$ have the meanings given previously and $R_3$ represents a

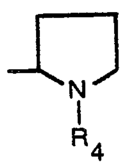 radical or 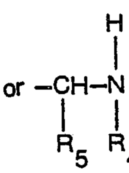 group,

in which $R_4$ represents a

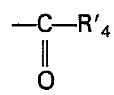 group,

$R'_4$ being an alkoyl radical containing from 1 to 5 carbon atoms, optionally substituted by a free or protected amino radical, a phenyl or benzyl radical, characterized in that a product of formula (I), in which $R_3$ represents a 2-pyrrolidinyl radical or a

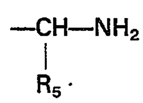 radical,

in the racemic or optically active form is reacted with a reactive derivative of the

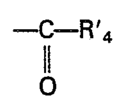 group,

so as to obtain a corresponding product of formula (I), in the racemic or optically active form, which is

subjected, if the case arises, to the action of a deblocking agent of the amino function, which product of formula (I), which if desired is subjected to the action of an acid to form the salt.

10. As medicaments, the products as defined in any one of claims 1 to 5, in their racemic or optically active forms, as well as their addition salts with pharmaceutically acceptable acids.

11. As medicaments, the products as defined in claim 6, in their racemic and optically active forms, as well as their addition salts with pharmaceutically acceptable acids.

12. As medicaments, the products as defined in claim 7, as well as their addition salts with pharmaceutically acceptable acids.

13. The pharmaceutical compositions containing as active principle, at least one of the medicaments as defined in claims 10 to 12.

14. As new industrial products, notably as intermediate products, the products of formula (VI), as defined in claim 8.

**Patentansprüche**

1. Verbindungen der Formel (I)

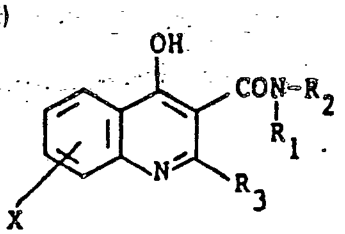

(I)

worin X in 5-, 6-, 7- oder 8-Stellung ein Wasserstoffatom, ein Halogenatom, einen linearen oder verzweigten Alkylrest mit 1 bis 5 Kohlenstoffatomen, einen linearen oder verzweigten Alkoxyrest mit 1 bis 4 Kohlenstoffatomen, eine Trifluormethylgruppe, eine Trifluormethylthiogruppe oder eine Trifluormethoxygruppe bedeutet, $R_1$ für ein Wasserstoffatom oder einen Alkylrest mit 1 bis 4 Kohlenstoffatomen steht, $R_2$ einen Rest wiedergibt, ausgewählt unter den Thiazolyl-, 4,5-Dihydrothiazolyl-, Pyridinyl-, Oxazolyl-, Isoxazolyl-, Imidazolyl-, Pyrimidyl-, und Tetrazolylresten, die gegebenenfalls durch eine Alkylgruppe mit 1 bis 4 Kohlenstoffatomen substituiert sind, und Phenyl, das gegebenenfalls durch einen oder mehrere Reste, ausgewählt unter der Hydroxygruppe, den Alkylresten mit 1 bis 4 Kohlenstoffatomen, den Alkoxyresten mit 1 bis 4 Kohlenstoffatomen, der Trifluormethylgruppe, der Nitrogruppe und den Halogenatomen, substituiert ist, $R_3$ für einen 2-Pyrrolidinylrest der Formel

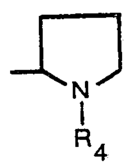

oder einen Rest de Formel

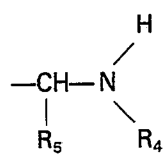

steht,

worin $R_4$ entweder ein Wasserstoffatom oder eine Gruppe

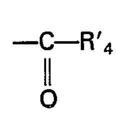

wiedergibt, wobei $R'_4$ einen Alkylrest mit 1 bis 5 Kohlenstoffatomen, der gegebenenfalls durch eine freie oder geschützte Aminogruppe substituiert ist, eine Phenyl- oder Benzylgruppe oder eine Schutzgruppe für die Aminofunktion bedeutet, $R_5$ für ein Wasserstoffatom, einen Alkylrest mit 1 bis 5 Kohlenstoffatomen, einen Phenyl- oder Benzylrest oder einen 4-OH-benzylrest, einen 1H-Indol-3-yl-methylrest der Formel

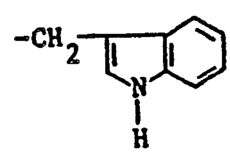

einen Rest

$-CH_2OH$ oder $-\underset{\displaystyle OH}{\underset{|}{CH}}-CH_3$ oder $-CH_2SH$ steht,

wobei die Alkohol- und Thiolfunktionen dieser drei letztgenannten Reste gegebenenfalls durch Schutzgruppen blockiert sind, in ihren racemischen oder optisch aktiven Formen sowie deren Additionssalze mit Säuren.

2. Verbindungen der Formel (I) gemäß Anspruch 1, worin sich X in 8-Stellung befindet und worin X für einen Trifluormethylrest steht, in deren racdemischen oder optisch aktiven Formen sowie deren Additionssalze mit Säuren.

3. Verbindungen der Formel (I) gemäß einem der Ansprüche 1 oder 2, worin $R_1$ für ein Wasserstoffatom steht und worin $R_2$ für einen Thiazolylrest steht, in deren racemischen oder optisch aktiven Formen sowie deren Additionssalze mit Säuren.

4. Verbindungen der Formel (I) gemäß den Ansprüche 1 bis 3, worin die Schutzgruppen für die Aminofunktion Gruppen der Formel $-CO_2A$ sind, worin A einen Alkylrest mit 1 bis 5 Kohlenstoffatomen, einen Aryl- oder Aralkylrest bedeutet, in deren racemischen oder optisch aktiven Formen sowie deren Additionssalze mit Säuren.

5. Verbindungen der Formel (I) gemäß den Ansprüche 1 bis 4, worin A für einen tert.-Butyl- oder Benzylrest steht, in deren racemischen oder optisch aktiven Formen sowie deren Additionssalze mit Säuren.

6. Verbindungen der Formel (I) gemäß einem der ansprüche 1 bis 3, worin $R_4$ ein Wasserstoffatom bedeutet, in deren racemischen oder optisch aktiven Formen sowie deren Additionssalze mit Säuren.

7. Verbindungen der Formel (I) gemäß einem der Ansprüche 1 bis 6 in deren optisch aktiver (S)-Form sowie deren Additionssalze mit Säuren.

8. Verfahren zur Herstellung der Produkte der Formel (I), worin X, $R_1$, $R_2$ und $R_3$ wie vorstehend definiert sind, dadurch gekennzeichnet, daß man eine Verbindung der Formel (II)

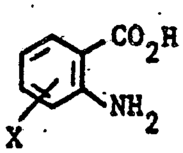 (II)

worin X die vorstehend angegebene Bedeutung besitzt, der Einwirkung einer Aminosäure der Formel (III)

$$R_3-COOH \qquad (III)$$

in aktivierter Form und in racemischer oder optisch aktiver Form untezieht, worin $R_3$ für eine Gruppe

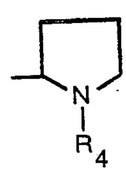

oder einen Gruppe

$-\underset{\displaystyle R_5}{\underset{|}{CH}}-N\genfrac{}{}{0pt}{}{\diagup H}{\diagdown R_4}$ steht,

worin $R_4$ entweder eine Gruppe

$-\underset{\displaystyle O}{\underset{\|}{C}}-R'_4,$

in der $R'_4$ die verstehend angegebene
Bedeutung besitzt, oder eine Schutzgruppe für die Aminofunktion ist, wobei $R_5$ die vorstehend angegebene Bedeutung hat, mit der Maßabe, daß, wenn $R_5$ eine Gruppe

$-CH_2OH$ oder $-CH(OH)-CH_3$ oder $-CH_2SH$

wiedergibt und wenn $R'_4$ für eine durch eine Aminogruppe substituierte Alkylgruppe steht, die Alkohol-, Thiol- und Amin-Funktionen gegebenenfalls blockiert sind, um zu einer Verbindung der Formel (IV) in racemischer oder optisch aktiver Form

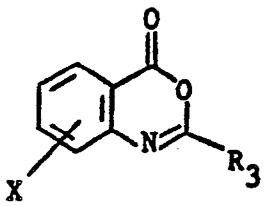

(IV)

zu gelangen, worin X und $R_3$ die vorstehend angegebenen Bedeutungen besitzen, die man in Gegenwart einer Base der Einwirkung einer Verbindung der Formel (V)

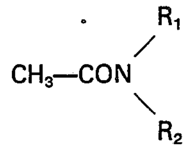

(V)

worin $R_1$ und $R_2$ die vorstehenden Bedeutungen besitzen, unterzieht, um zu einer Verbindung der Formel (VI) in racemischer oder optisch aktiver Form

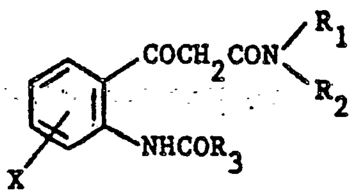

(VI)

zu gelangen, worin X, $R_1$, $R_2$ und $R_3$ die vorstehenden Bedeutungen besitzen, die man in Anwesenheit eines alkalischen Mittels cyclisiert, um eine Verbindung der Formel (I), worin X, $R_1$, $R_2$ und $R_3$ die angegebenen Bedeutungen besitzen, in racemischer oder optisch aktiver Form zu erhalten, die man gegebenenfalls der Einwirkung eines oder mehrerer Mittel für die Abspaltung von Schutzgruppen für die Amino-, Alkohol- und Thiol-Funktionen unterzieht, um zu einem Produkt der Formel (I) zu gelangen, worin X, $R_1$ und $R_2$ die vorstehenden Bedeutungen besitzen und worin $R_3$ entweder eine Gruppe

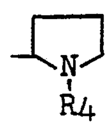 oder eine Gruppe 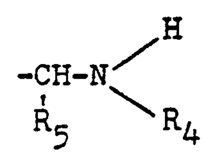

wiedergibt, worin $R_4$ für eine Gruppe

$-C(=O)-R'_4$ oder ein

Wasserstoffatom steht und $R_5$ die vorstehende Bedeutung besitzt, mit der Maßgabe, daß, wenn $R_5$ eine Gruppe

$CH_2OH$, $CH(OH)-CH_3$ oder $CH_2SH$

bedeutet und wenn $R'_4$ einen durch eine Aminogruppe substituierten Alkylrest wiedergibt, die Alkohol-, Thiol- und Amino-Funktionen frei sind, und die Produkte der Formel (I) in racemischer oder optisch aktiver Form gewünschtenfalls der Einwirkung einer Säure unterzieht, um hieraus das Salz zu bilden.

9. Verfahren zur Herstellung der Produkte der Formel (I) gemäß Anspruch 1, worin X, $R_1$ und $R_2$ die angegebenen Bedeutungen besitzen und $R_3$ einen Rest

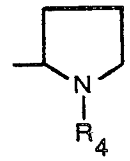

oder 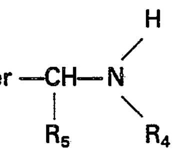

bedeutet, worin $R_4$ eine Gruppe

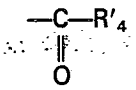

wiedergibt, wobei $R'_4$ eine Alkylgruppe mit 1 bis 5 Kohlenstoffatomen, die gegebenenfalls durch eine freie oder geschützte Aminogruppe substituiert ist, einen Phenyhl- oder Benzylrest bedeutet, dadurch gekennzeichnet, daß man ein Produkt der Formel (I), worin $R_3$ einen 2-Pyprrolidinylrest ode einen Rest

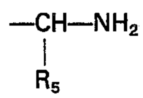

bedeutet, in racemischer oder optisch aktiver Form mit einem reaktiven Derivat der Gruppe

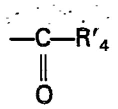

umsetzt, um zu einem entsprechenden Produkt der Formel (I) in racemischer oder optisch aktiver Form zu gelangen, das man gegebenenfalls der Einwirkung eines Mittels zur Entfernung der Schutzgruppe der Aminofunktion unterzieht, und das Produkt der Formel (I) gewünschtenfalls der Einwirkung einer Säure unterzieht, um hieraus das Salz zu bilden.

10. Als Arzneimittel die Produkte gemäß einem der Ansprüche 1 bis 5 in ihren racemischen oder optisch aktiven Formen sowie deren Additionssalze mit pharmazeutisch verträglichen Säuren.

11. Als Arzneimittel die Produkte gemäß Anspruch 6 in ihren racemischen oder optisch aktiven Formen sowie deren Additionssalze mit pharmazeutisch verträglichen Säuren.

12. Als Arzneimittel die Produkte gemäß Anspruch 7 sowie deren Additionssalze mit pharmazeutisch verträglichen Säuren.

13. Pharmazeutische Zusammensetzungen, enthaltend als Wirkstoff zumindest eines der Arzneimittel gemäß einem der Ansprüche 10 bis 12.

14. Als neue, industrielle Produkte, insbesondere als Zwischenprodukte, die Produkte der Formel (VI) gemäß Anspruch 8.